# EUROPEAN PATENT SPECIFICATION

(11) **EP 4 310 494 B1**
(45) Date of publication and mention of the grant of the patent: **08.07.2026**
(21) Application number: 21931678.3
(22) Date of filing: 05.10.2021
(51) Int. Cl.: G01N 30/86, G01N 30/72

(54) **LEARNING DATA CREATION METHOD AND LEARNING DATA CREATION DEVICE**
LERNDATENERZEUGUNGSVERFAHREN UND LERNDATENERZEUGUNGSVORRICHTUNG
PROCÉDÉ DE CRÉATION DE DONNÉES D'APPRENTISSAGE ET DISPOSITIF DE CRÉATION DE DONNÉES D'APPRENTISSAGE

(30) Priority: 19.03.2021 US 202163163205 P
(43) Date of publication of application: 24.01.2024
(73) Proprietor: Shimadzu Corporation, Nakagyo-ku, Kyoto-shi, Kyoto 604-8511 (JP)
(72) Inventor: SAKAI, Takero, Columbia, Maryland 21046 (US)
(74) Representative: Müller-Boré & Partner Patentanwälte PartG mbB
(86) International application number: PCT/JP2021/036791
(87) International publication number: WO 2022/195935

(56) References cited:
- WO-A1-2020/044435
- WO-A1-2020/070786
- WO-A1-2020/070786
- JP-A- 2009 204 397
- JP-A- 2017 122 677
- JP-A- 2019 056 664
- JP-A- 2019 056 664
- JP-A- 2019 086 475
- JP-A- 2020 165 847
- ANONYMOUS: "Analysis sequence", 20 March 2015 (2015-03-20), XP055967952, Retrieved from the Internet <URL:www.lcdez.com/_userdata/Support/ChromatoStage/Help/%E6%B3%A2%E5%BD%A2%E5%87%A6%E7%90%86%E3%81%AE%E5%8E%9F%E7%90%86%EF%BC%8D%E8%A7%A3%E6%9E%90%E3%82%B7%E3%83%BC%E3%82%B1%E3%83%B3%E3%82%B9.htm> [retrieved on 20221004]

## Description

### TECHNICAL FIELD

The present invention relates to a technique of creating learning data from measurement data acquired by measuring samples using a sample analyzer such as a chromatograph mass spectrometer.

### BACKGROUND ART

High-level quality control is required in the processes, such as production, processing and distribution, of foods, and demands for the quality control have been further increasing in recent years. Conventionally, degradation of food quality in the production and processing sites is generally evaluated based on human's subjective sense such as color, odor, and taste. On the other hand, quality evaluation using a sample analyzer has been attempted in order to perform more objective and efficient evaluation. For example, Patent Literature 1 reports that the freshness of fish meat is evaluated by quantitatively analyzing non-volatile biological amines generated when fish meat decays.

However, a food sample contains a wide variety of substances, and it may not be possible to sufficiently grasp a change in quality only by analyzing the concentration of an indicator substance. Furthermore, in many cases, even foods that are identical to each other in type and freshness have different contents of substances individually, and there is a problem in that erroneous determination or overlooking easily occurs particularly when the degree of progress of deterioration of the food is low.

Thus, it has been attempted to construct a learning model by setting, as learning data, measurement data acquired by measuring, with a specific sample analyzer, each of a plurality of reference samples (food samples or the like) having known label information (freshness or the like) indicating the attribute of a target object, and by performing machine learning using the learning data. In this learning model construction method, machine learning is performed on measurement data of an enough number of reference samples so that a parameter or parameters of the learning model converge to a level at which the freshness of a target sample can be discriminated with accuracy higher than a predetermined standard , and the converged parameter or parameters are used as a discriminator. The discriminator thus created determines a correlation of a feature amount (for example, positions and intensities of peaks in measurement intensity, which are derived from a plurality of substances characteristic of a reference sample (of the same freshness) having the same label information) common to learning data having the same label information, and discriminates the freshness of the target sample. Therefore, erroneous determination and overlooking are less likely to occur than analysis based only on a single or specific type of indicator substance. WO2020/044435 A1 describes a method of analyzing data by setting a value for each of one or more analysis parameters and by using a predetermined analysis program. WO2020/070786 A1 describes a method of generating a discriminator for performing peak detection, comprising a step of obtaining first waveform data having a first peak and a second peak having a peak position different from the first peak.
JP 2019 056664 A describes an odor determination system and an odor determination program capable of increasing the quality of a determinable odor more than a conventional one.

### CITATION LIST

### PATENT LITERATURE

Patent Literature 1: JP 2017-122677 A
Patent Literature 2: JP 2020-165847 A

### SUMMARY OF INVENTION

### TECHNICAL PROBLEM

In order to create a discriminator that accurately discriminates the freshness of a target sample, it is necessary to construct a learning model by performing machine learning using a large number of learning data in which feature amounts included in the measurement data of a reference sample are correctly extracted. For example, when measurement data (mass chromatogram) acquired through gas chromatograph mass spectrometry is to be used, learning data is created by extracting feature amounts (retention time, mass-to-charge ratio, and measurement intensity) corresponding to each of all peaks derived from substances contained in a reference sample.

When extracting a peak from a mass chromatogram (i.e. peak picking), the user selects a method suitable for the shape of a baseline in the chromatogram from a plurality of methods (combinations of an algorithm for extracting a peak and a parameter such as a threshold related to a peak height) prepared in advance. Even when reference samples have the same label information, the amounts of substances contained in the reference samples are not the same. Furthermore, a large number of reference samples may be measured a plurality of times or may be measured by a plurality of devices. In this case, even when the amounts of substances contained in each of the reference samples are the same, the shape of a baseline and the height of a peak in the measurement data are slightly different for each of the samples. Therefore, even if peak picking is performed using the same method and parameter, there is a case where a small peak included in the measurement data of some reference samples cannot be extracted, and such learning data is created that a feature amount corresponding to the peak that has not been extracted is defective. When learning data in which a feature amount is defective is used, it is difficult to cause a parameter of the learning model to converge. Furthermore, even though the parameter of the learning model is caused to converge, using such a learning model as a discriminator may lower the accuracy of discrimination. It is possible to exclude measurement data from which some of the peaks have not been extracted and create learning data only from measurement data from which all of the peaks have been extracted. However, in this case, since the number of learning data is reduced by the number of measurement data excluded, there is also a possibility that it is difficult to cause a parameter of a learning model to converge or the accuracy of a discriminator may be deteriorated.

An object of the present invention is to provide a technique capable of creating learning data in which deficiency in the feature amount is suppressed when a peak is extracted from measurement data acquired by analyzing a reference sample having a known sample attribute to create learning data.

### SOLUTION TO PROBLEM

The present invention made to solve the above problems is directed to a learning data creation method for creating learning data to be used for creating a discriminator that discriminates a plurality of target samples having attributes that differ from each other. The present invention is defined by the independent claims. Preferred examples are defined in the dependent claims.

### ADVANTAGEOUS EFFECTS OF INVENTION

The attribute described above is, for example, freshness or a production area when a reference sample is a food sample, and is, for example, presence or absence of a specific disease when a reference sample is a biological sample.

In the learning data creation method according to the present invention, mass chromatogram data through measurement using a chromatograph mass spectrometer is acquired for each of a plurality of samples having known attributes. Furthermore, in the learning data creation device according to the present invention, mass chromatogram data is acquired, as measurement data, through measurement using a chromatograph mass spectrometer for each of a plurality of reference samples having known attributes. The measurement data may be acquired through actual measurement of a sample with a sample analyzer or through reading of measurement data acquired through measurement in advance.

Next, for the measurement data of each of the plurality of samples, a peak in measurement intensity is extracted using the first method prepared in advance. Then, it is determined whether or not all peaks are extracted (i.e. presence or absence of a peak) by collating the extracted peaks with peak information which is prepared in advance of a standard sample which has a same attribute as the reference sample. As the peak information of the standard sample, for example, information stored in a library can be used. Alternatively, it is also possible to integrate measurement data of a plurality of reference samples having a same attribute to create integrated measurement data, and use peak information extracted from the integrated measurement data using the first method as peak information of the standard sample. The first method and the second method both use a combination of an algorithm for extracting a peak and a parameter such as a threshold related to a peak height. As an algorithm for extracting a peak, a conventionally used algorithm may be used, or a characteristic algorithm described in embodiments described later may be used.

For measurement data of a sample, which is determined to have absence of a peak, a peak in measurement intensity is extracted using the second method that differs from the first method in an algorithm or a parameter for extracting a peak. Then, presence or absence of the peak is determined by collating the peak extracted using the second method with the peak information which is prepared in advance of the standard sample which has a same attribute as the reference sample. Finally, a feature amount corresponding to each of the plurality of peaks in the measurement data is acquired from the measurement data determined that there is no absence of the peak extracted using the first method or the second method to create learning data.

In the learning data creation method and the learning data creation device according to the present invention, learning data is created by acquiring a feature amount corresponding to a peak in measurement intensity in measurement data from the measurement data determined to have no absence of a peak. It is therefore possible to create learning data in which deficiency in feature amount is suppressed. Furthermore, even in a case of measurement data from which all peaks cannot be extracted using the first method, a feature amount is acquired from the measurement data from which all peaks can be extracted using the second method, to create learning data, so that a decrease in the number of learning data can also be suppressed.

### BRIEF DESCRIPTION OF DRAWINGS

Fig. 1 is a configuration diagram of a main part of a sample evaluation system including one embodiment of a learning data creation device according to the present invention.
Fig. 2 is a flowchart related to one embodiment of a learning data creation method according to the present invention.
Fig. 3 is a schematic diagram of mass chromatogram data representing measurement data acquired in the present embodiment.
Fig. 4 is a schematic diagram related to a conventional peak extraction method.
Fig. 5 is an example result when peaks are extracted from a mass chromatogram with the conventional peak extraction method.
Fig. 6 is another example result when peaks are extracted from a mass chromatogram with the conventional peak extraction method.
Fig. 7 is still another example result when peaks are extracted from a mass chromatogram with the conventional peak extraction method.
Fig. 8 is a schematic diagram related to defining of a baseline with a peak extraction method according to the present embodiment.
Fig. 9 is an example where a baseline is defined in a mass chromatogram with the peak extraction method according to the present embodiment.
Fig. 10 is an example result when peaks in a mass chromatogram are extracted with the peak extraction method according to the present embodiment.

### DESCRIPTION OF EMBODIMENTS

One embodiment of a learning data creation method and a learning data creation device according to the present invention will be described below with reference to the drawings.

A sample evaluation system 1 according to the present embodiment is used to estimate an attribute of a target sample. Specifically, it is used to estimate the freshness and production area of a target sample, an example of which is a food, or to determine whether or not a subject has a specific disease based on a biological sample.

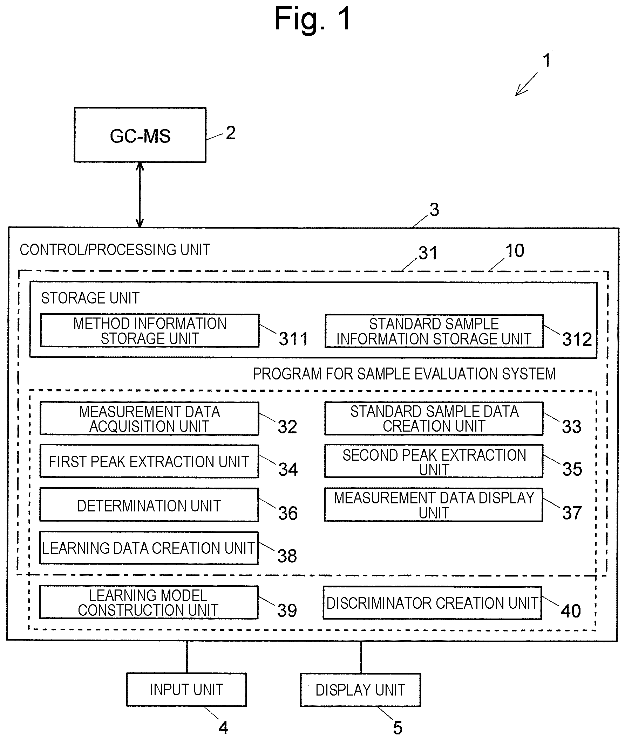

Fig. 1 illustrates a configuration of a main part of the sample evaluation system 1 including the learning data creation device according to the present embodiment. The sample evaluation system 1 according to the present embodiment roughly includes a gas chromatograph mass spectrometer (GC-MS) 2 and a control/processing unit 3.

The control/processing unit 3 includes a storage unit 31. The storage unit 31 is provided with a method information storage unit 311 and a standard sample information storage unit 312. The method information storage unit 311 stores information of methods used for extracting a peak from mass chromatogram data acquired through measurement of a sample using the gas chromatograph mass spectrometer 2. The information of the methods includes information related to combinations of an algorithm for extracting a peak and a parameter such as a threshold related to a peak height. The algorithm for extracting a peak may include a link point method, a horizontal method, and a new baseline method, which are used conventionally, and a method using a minimum measurement point, which is characteristic in the present embodiment (described later).

The standard sample information storage unit 312 stores, for various samples, mass chromatogram data of a plurality of standard samples having different attributes and peak information (retention time, mass-to-charge ratio, and measurement intensity) related to a plurality of peaks appearing in the mass chromatogram. The storage unit 312 additionally stores measurement conditions and the like used when various samples are measured using the gas chromatograph mass spectrometer 2.

The control/processing unit 3 further includes, as functional blocks, a measurement data acquisition unit 32, a standard sample data creation unit 33, a first peak extraction unit 34, a second peak extraction unit 35, a determination unit 36, a measurement data display unit 37, a learning data creation unit 38, a learning model construction unit 39, and a discriminator creation unit 40. An entity of the control/processing unit 3 may be a personal computer or a higher-performance computer called a workstation, and each of the functional blocks described above is embodied by causing a processor of the computer to execute a program for the sample evaluation system, which is installed in advance in the computer. Furthermore, the control/processing unit 3 is connected with an input unit 4 such as a keyboard and a mouse and a display unit 5 such as a liquid crystal display.

Among components of the control/processing unit 3, the functional blocks of the method information storage unit 311 and the standard sample information storage unit 312 and the functional blocks of the measurement data acquisition unit 32, the standard sample data creation unit 33, the first peak extraction unit 34, the second peak extraction unit 35, the determination unit 36, the measurement data display unit 36, and the learning data creation unit 38 configure the learning data creation device 10 according to the present embodiment. In the present embodiment, the learning data creation device 10 is incorporated in a part of the control/processing unit 3. However, the learning data creation device 10 may be configured as a device independent of the control/processing unit 3.

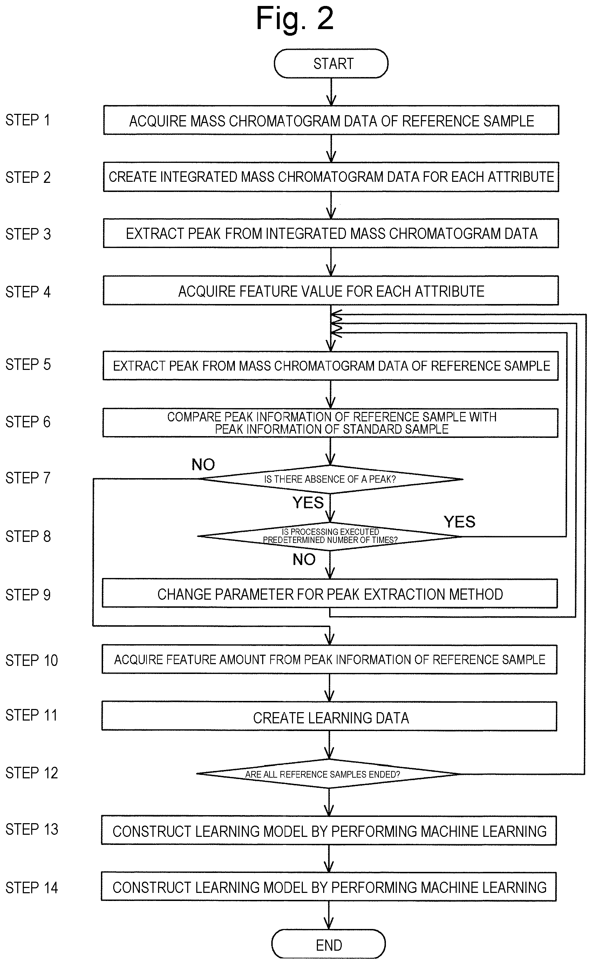

Next, the operation of the sample evaluation system 1 according to the present embodiment will be described with reference to the flowchart of Fig. 2. In this example, a discriminator for estimating the attribute of a target sample is created.

Prior to creating a discriminator, a user sets a reference sample in an autosampler (not illustrated) connected to the gas chromatograph mass spectrometer 2. The reference sample is identical in type to a target sample, and has a known attribute. Furthermore, a plurality of the reference samples are set for each attribute.

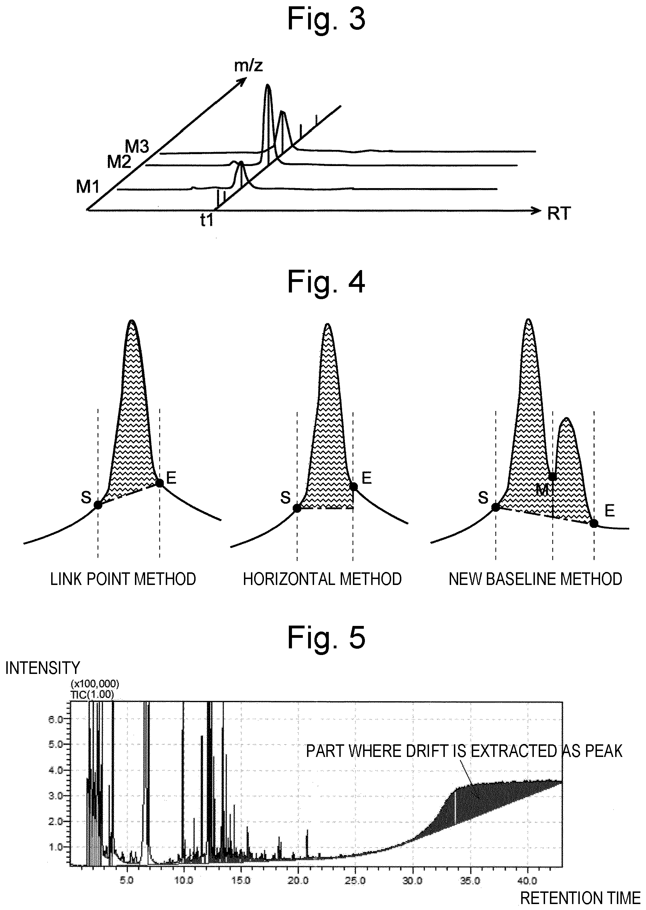

When the user instructs start of creation of a discriminator, the measurement data acquisition unit 32 introduces the reference samples set in the autosampler into the gas chromatograph mass spectrometer 2 in a predetermined order. In the gas chromatograph mass spectrometer 2, components are separated from each other in a column of the gas chromatograph. The components are then introduced into the mass spectrometer. The components are each ionized by an ionization source such as an electron ionization source, mass-separated, and detected by an ion detector. Output signals from the ion detector are sequentially sent to the control/processing unit 3. Measurement data (mass chromatogram data) of each of the reference samples is stored in the storage unit 31. The mass chromatogram data is thus acquired for all the reference samples (step 1). As schematically illustrated in Fig. 3, the mass chromatogram data represents measurement intensities of ions with respect to two parameters of time and mass-to-charge ratio, and has peaks in the mass-to-charge ratios of the ions generated from the components contained in each of the samples at the time (retention time around t1) when the components flow out of the column of the gas chromatogram.

When measurement of all the reference samples is completed and the mass chromatogram data is stored, the standard sample data creation unit 33 groups the reference samples for each attribute. Then, the mass chromatogram data of the reference samples having a same attribute are integrated to create integrated mass chromatogram data (step 2). The standard sample data creation unit 33 then activates the first peak extraction unit 34 to create an extraction ion current chromatogram (chromatogram of the measurement intensities of the ions having a specific mass-to-charge ratio) from the integrated mass chromatogram data, and extracts a peak using an algorithm and a parameter stored in the method information storage unit 311 (step 3).

Here, processing of extracting a peak from the extraction ion current chromatogram by the first peak extraction unit 34 will be described. Conventionally, for example, the link point method, the horizontal method, or the like is used as a method for extracting a peak from an extraction ion current chromatogram or a total ion current chromatogram indicating the intensities of all ions (Fig. 4). In Fig. 4, peaks extracted using the respective methods are hatched. In the link point method, a point at which the slope of the waveform of a chromatogram exceeds a value determined in advance is defined as a peak start point S. A point at which the slope of the waveform falls after that below the value determined in advance is defined as a peak end point E. Then, a baseline is defined by connecting the peak start point S and the peak end point E. A peak is thus extracted. Furthermore, in the horizontal method, after a peak start point S and a peak end point E are defined in the same manner as described above, a horizontal line passing through one of the two points, at which the measurement intensity is lower than that of the other point, to an intersection with a perpendicular from the other point is defined as a baseline. A peak is thus extracted. Furthermore, for example, the new baseline method is used to separate a plurality of peaks superimposed on each other. In the new baseline method, a peak start point S and a peak end point E are defined in the same manner as described above. Peaks are separated using a minimum point located between the two peaks as a peak separation point M.

In a case of measurement data with little variation in a baseline, there is no significant difference in the accuracy of peak extraction regardless of whether the link point method or the horizontal method is used. However, in a case of measurement data acquired through measurement using a gas chromatograph, a baseline may rise as time passes by in the second half of a chromatogram due to a phenomenon called column bleed. Conventionally, a peak is automatically extracted from chromatogram data using a preset algorithm such as the link point method, the horizontal method, or the new baseline method. However, when the set algorithm or parameter is not appropriate, a rise of a baseline due to drift in the second half of a chromatogram is extracted as a peak, as illustrated in Fig. 5.

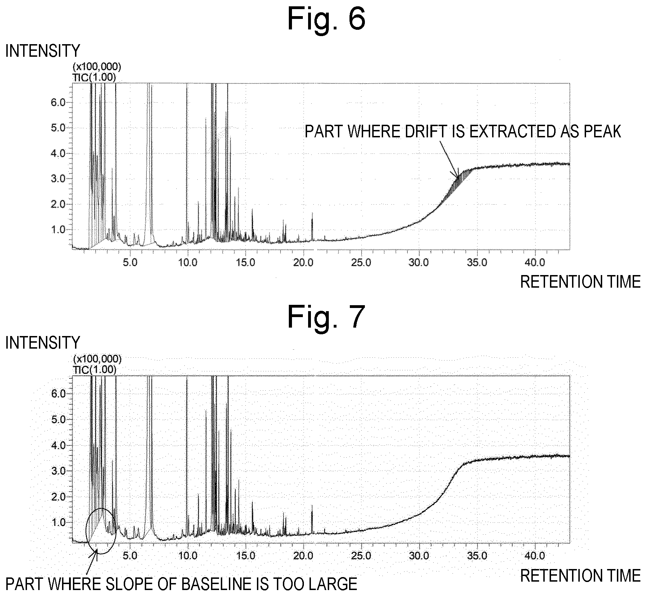

In order to address such drift, it may be possible to set a parameter that takes into account a rise of a baseline due to drift. In Fig. 6, this parameter is set to a value of 50. However, even when this parameter value is set, a rise of a baseline due to drift is still extracted as a peak. In Fig. 7, this parameter is set to a value of 100. In Fig. 7, no drift is extracted as a peak, which is deemed to be appropriate in this respect. However, the slope of the baseline is too large in the first half of the chromatogram. It is thus difficult to correctly acquire the height and area of a peak.

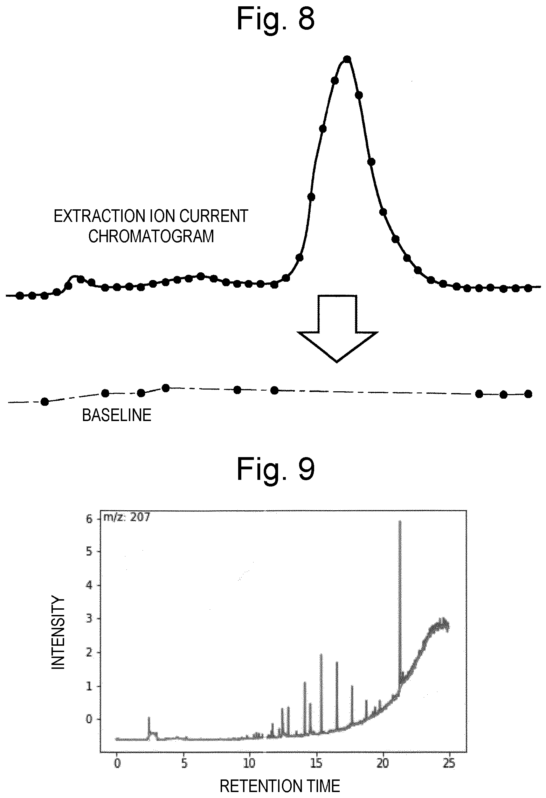

Thus, in the present embodiment, minimum points on a chromatogram waveform are connected to define a baseline. A peak is thus extracted. As schematically illustrated in the upper part of Fig. 8, an acquired chromatogram is one where a large number of measurement points are connected. The algorithm according to the present embodiment extracts, as a minimum measurement point, one measurement point with a smaller measurement intensity than those of two adjacent measurement points, among the measurement points forming the chromatogram. Then, the extracted minimum measurement points are linearly interpolated to define a baseline as illustrated in the lower part of Fig. 8. Note that the method for defining a baseline from minimum measurement points is not limited to linear interpolation. An approximate curve or the like connecting minimum measurement points may be used. Then, one having a height from the baseline exceeding a threshold determined in advance is extracted as a peak.

In an extraction ion current chromatogram, superimposing of peaks does not occur unless both of the two parameters of retention time and mass-to-charge ratio are common to each other. Therefore, a baseline may be defined using linear interpolation. However, in a chromatogram (total ion current chromatogram or the like) where there is one parameter for measurement intensity, superimposed peaks may appear. In this case, a minimum point may appear between the two peaks. When minimum measurement points including minimum points between peaks are linearly interpolated in such a case, a baseline cannot be correctly defined. In such a case, an approximate curve for all the minimum measurement points is acquired, instead of linearly interpolating the minimum measurement points, for example, to correctly define a baseline, while reducing the influence of outliers such as minimum points between superimposed peaks. Alternatively, an analyst may delete outliers such as minimum points between peaks and linearly interpolate other minimum measurement points to define a baseline.

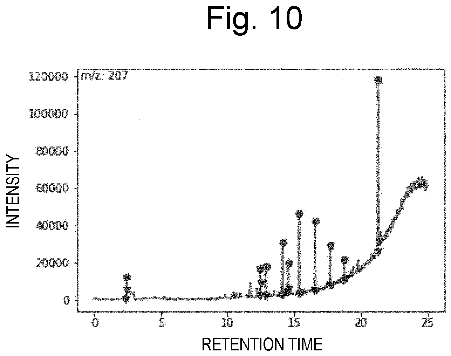

Fig. 9 is a diagram illustrating a result of defining a baseline for mass chromatogram data in which there is a large drift in the second half, similarly to the chromatograms illustrated in Figs. 5 to 7. Fig. 10 is a diagram illustrating a result of extracting peaks (circles in the diagram illustrate peak tops) higher than a threshold determined in advance to be higher than the baseline serving as a standard. As can be seen from this result, even when there is a large drift in the second half of the chromatogram, the peaks can be correctly extracted using the method according to the present embodiment.

Even when any of mass chromatogram data of a plurality of reference samples having a same attribute include peaks that are difficult to extract, integrating the mass chromatogram data achieves compensation by the measurement intensities of the other reference samples. Extraction of a peak is thus facilitated. Therefore, when integrated mass chromatogram data is used, all peaks can be easily extracted. Thus, in the present embodiment, position information of a plurality of peaks extracted from integrated mass chromatogram data is used as peak information of a standard sample. The standard sample data creation unit 33 causes the standard sample information storage unit 312 to store the peak information of the standard sample for each attribute.

Next, the first peak extraction unit 34 extracts peaks from mass chromatogram data of each reference sample using an algorithm and a parameter stored in the method information storage unit 311 in the same manner as described above (step 5).

When peaks are extracted by the first peak extraction unit 34, the determination unit 36 reads the peak information of the standard sample which has a same attribute as the reference sample from the standard sample information storage unit 312. Then, peak information of the reference sample and the peak information of the standard sample are compared with each other (step 6).

When the determination unit 36 determines that the peak information of the reference sample and the peak information of the standard sample coincide with each other (i.e., there is no absence of a peak) (NO in step 7), feature amounts corresponding to the peaks extracted from the mass chromatogram data of the reference sample are acquired to create learning data (step 11). Here, a feature amount to be extracted includes a set of a retention time and a mass-to-charge ratio corresponding to the peak top of each peak. Furthermore, a height or area value of the peak may be included in the feature amount in addition to those described above. That is, one corresponding to a peak list is created as learning data.

When the determination unit 36 determines that the peak information of the reference sample and the peak information of the standard sample do not coincide with each other (one or more of a plurality of peaks that should be extracted is or are not extracted) (YES in step 7), a screen is caused to be displayed to notify that one or more peaks are absent for the reference sample. Furthermore, the measurement data display unit 37 creates an extraction ion current chromatogram corresponding to the mass-to-charge ratio(s) of the one or more peaks that is or are absent in the mass chromatogram data of the reference sample and causes the screen of the display unit 5 to display the created extraction ion current chromatogram. In the mass chromatogram displayed on the screen, a mark is displayed in a superimposed manner at a position (i.e., position of the retention time and the mass-to-charge ratio of the peak that is included in the peak information of the standard sample and is not included in the peak information of the reference sample) where no peak has been extracted by the first peak extraction unit 34 although there is the peak that should be extracted. The user can check the waveform of the extraction ion current chromatogram at the position where the mark is displayed, and determine whether or not the second peak extraction unit 35 can extract the one or more peaks by changing the algorithm or the parameter.

Furthermore, the determination unit 36 determines whether or not the number of times of performing the processing of extracting a peak on the reference sample has reached the number of times determined in advance (predetermined number of times). The number of times is set to five, for example. At this stage, since the first peak extraction unit 34 has only performed the extraction of a peak (the number of peak extraction processing is 1), it is determined that the predetermined number of times has not been reached (NO in step 8).

When NO is determined in step 8, the second peak extraction unit 35 changes the algorithm and/or the parameter used at the time of the peak extraction performed previously (step 9). In this example, the algorithm is not changed, and the threshold (the height of the baseline) used to determine a peak is lowered. Specifically, for example, a threshold (parameter) at the time of the peak extraction is changed to 90% of the threshold at the time of the peak extraction by the first peak extraction unit 34 (the threshold is lowered by 10%).

Then, the second peak extraction unit 35 uses the threshold after changed to again extract a peak from the mass chromatogram data of the reference sample (step 5). Then, comparison with the peak information of the standard sample is performed again (step 6). When it is determined that there is no absence of a peak (NO in step 7), a feature amount corresponding to each of the extracted plurality of peaks is acquired to create learning data (step 11). After the learning data is created, it is determined whether or not the processing for the mass chromatogram data of all the reference samples has been completed (step 12). Then, when unprocessed data is present, the mass chromatogram data of the next reference sample is subjected to step 5 and later steps in the order as described above.

On the other hand, when it is determined that there is absence of a peak again (YES in step 7), it is determined whether or not the number of times of the peak extraction processing has reached the predetermined number of times (step 8). When it is determined that the predetermined number of times has not reached, the algorithm and/or the parameter is or are changed again (step 9). In this example, the threshold at the time of the peak extraction processing performed by the first peak extraction unit 34 is regarded as 100, and the threshold is lowered by 10 in a stepwise manner. Alternatively, the threshold may be lowered by 10% in a stepwise manner based on the time of the previous peak extraction processing.

When the absence of the peak has not yet been solved even after performing the processing of extracting a peak the predetermined number of times (YES in step 8), the processing related to the mass chromatogram data of the reference sample is ended. Step 5 and later steps are then performed on the mass chromatogram data of the next reference sample in the order as described above.

When the processing on the mass chromatogram data of all the reference samples is completed (YES in step 12), the learning model construction unit 39 constructs a learning model by performing machine learning using the learning data created in the procedure described above (step 13). As a method for performing machine learning, for example, a method called supervised learning can be used. Specifically, in addition to a representative machine learning method such as a support vector machine, a neural network, or a random forest, a multivariate analysis method such as logistic regression, an orthogonal partial least squares method, or a k-nearest neighbor method can be used. When the learning model is constructed by performing machine learning, the discriminator creation unit 40 creates a discriminator using the learning model and stores the created discriminator in the storage unit 31 (step 14).

Conventionally, in creating learning data, when a peak extracted from mass chromatogram data of a reference sample having a known attribute is absent, for example, the mass chromatogram data is excluded, and learning data is created using only those from which all peaks are extracted. As a result, the number of earning data is reduced by the number of mass chromatogram data excluded. Thus, it may be difficult to cause a parameter of a learning model to converge or the accuracy of a discriminator may be deteriorated. Alternatively, in Patent Literature 2, learning data is created by deleting all information of peaks that do not appear in common in all learning data (there is absence of a peak in any of learning data). In this case, such learning data is created that information of a peak, in which the peak is likely to be useful for determining an attribute, is deleted. There is also a possibility that it is difficult to cause a parameter of the learning model to converge or the accuracy of a discriminator may be deteriorated.

On the other hand, in the present embodiment, even when a peak is absent in the peak extraction processing performed by the first peak extraction unit 34, the second peak extraction unit 35 attempts to extract the peak a predetermined number of times, while changing an algorithm or a parameter. Therefore, a decrease in the number of learning data can be suppressed. Furthermore, since learning data is created after it is confirmed that all peaks are extracted through comparison with the peak information of the standard sample, it is possible to create the learning data where there is no deficiency in feature amount.

The above-described embodiment is merely an example. The numerical values described in the embodiment described above are merely examples, and can be changed as appropriate according to the characteristics of a target sample and measurement data and the like.

Furthermore, in the embodiment described above, the user may check a mass chromatogram determined to have absence of a peak on the screen of the display unit 5. The user may provide the second peak extraction unit 35 with an instruction for extracting the peak only when it is confirmed that there is a peak at a position at which a peak is not extracted. With this configuration, it is possible to efficiently create learning data without taking time to process mass chromatogram data from which peak extraction is impossible (or extremely difficult).

In the embodiment described above, only the algorithm of defining a baseline from minimum measurement points and of extracting a peak is used, and only the threshold is changed when a peak is extracted by the second peak extraction unit 35. However, the algorithm of extracting a peak may be changed when a peak is extracted by the second peak extraction unit 35.

In the embodiment described above, mass chromatogram data acquired by measuring a reference sample by the gas chromatograph mass spectrometer 2 is processed. However, a similar configuration to that in the present embodiment can be applied to various kinds of measurement data including a plurality of peaks in measurement intensity with respect to a predetermined parameter. Note that peaks referred to herein may include downward peaks appearing in an absorption spectrum or the like.

With the learning data creation method described above, measurement data including a plurality of peaks in measurement intensity with respect to a predetermined parameter is first acquired for each of a plurality of samples having known attributes. Furthermore, with the learning data creation device described above, mass chromatogram data is acquired, as measurement data, through measurement using a chromatograph mass spectrometer for each of a plurality of reference samples having known attributes. The measurement data may be acquired through actual measurement of a sample with a sample analyzer or through reading of measurement data acquired through measurement in advance.

Next, for the measurement data of each of the plurality of samples, a peak in measurement intensity is extracted using the first method prepared in advance. Then, it is determined whether or not all peaks are extracted (presence or absence of a peak) by collating the extracted peaks with peak information which is prepared in advance of a standard sample which has a same attribute as the reference sample. As the peak information of the standard sample, for example, information stored in a library can be used.

For measurement data of a sample, which is determined to have absence of peak, a peak in measurement intensity is extracted using the second method that differs from the first method in an algorithm or a parameter for extracting a peak. Then, presence or absence of the peak is determined by collating the peak extracted using the second method with the peak information which is prepared in advance of the standard sample which has a same attribute as the reference sample. Finally, a feature amount corresponding to each of the plurality of peaks in the measurement data is acquired from the measurement data determined that there is no absence of the peak extracted using the first method or the second method to create learning data.

With the learning data creation method described above and the learning data creation device described above, learning data is created by acquiring a feature amount corresponding to a peak in measurement intensity in measurement data from the measurement data determined to have no absence of a peak. It is therefore possible to create learning data in which deficiency in feature amount is suppressed. Furthermore, even in a case of measurement data from which all peaks cannot be extracted using the first method, a feature amount is acquired from the measurement data from which all peaks can be extracted using the second method, to create the learning data, so that a decrease in the number of learning data can be suppressed.

With the learning data creation method described above, it is possible to confirm whether or not there is an extractable intensity peak by the user confirming, on the screen, the measurement data determined to have absence of a peak. Furthermore, with the learning data creation method described above, by the user designating only the measurement data in which an extractable intensity peak exists, the burden of the processing related to the extraction of peak information using the second method can be reduced.

With the learning data creation method described above, the peak information of the standard sample can also be used for a sample for which a sufficient number of measurements have not been performed in the past and a sample for which measurement data is not recorded in a database or the like, such as an unknown sample.

With the learning data creation method described above, since the processing of automatically creating learning data is repeatedly performed a predetermined number of times, it is not necessary for the user to determine the necessity of the processing of collecting peak information each time, and the burden on the user is reduced.

In mass chromatogram data acquired through gas chromatograph mass spectrometry, the magnitude of a baseline also changes as time passes by. With the learning data creation method described above, since a baseline is determined using minimum measurement point, it can be suitably used to extract a peak from measurement data in which not only the measurement intensity but also the baseline changes with respect to the parameter. Furthermore, when peaks are extracted from such measurement data, a method in which only a threshold used for determining whether or not a peak is present is changed from that of the first method can be used as the second method, as in the learning data creation method described above.

### REFERENCE SIGNS LIST

1... Sample Evaluation System
10... Learning Data Creation Device
2... Gas Chromatograph Mass Spectrometer
3... Control/Processing Unit
31... Storage Unit
311... Method Information Storage Unit
312... Standard Sample Information Storage Unit
32... Measurement Data Acquisition Unit
33... Standard Sample Data Creation Unit
34... First Peak Extraction Unit
35... Second Peak Extraction Unit
36... Determination Unit
37... Measurement Data Display Unit
38... Learning Data Creation Unit
39... Learning Model Construction Unit
39... Discriminator Creation Unit
4... Input Unit
5... Display Unit

## Claims

1. A learning data creation method for creating learning data to be used for creating a discriminator that discriminates a plurality of target samples having attributes that differ from each other based on a feature amount with respect to a peak included in mass chromatogram of each target sample, the method comprising performing by a computer following steps of:
acquiring mass chromatogram data through measurement using a chromatograph mass spectrometer for each of a plurality of reference samples having known attributes;
extracting peak information related to the plurality of peaks using a first method prepared in advance for the mass chromatogram data of each of the plurality of reference samples;
determining absence of a peak by collating the peak information extracted using the first method with peak information which is prepared in advance of a standard sample which has a same attribute as each of the plurality of reference samples;
extracting peak information related to the plurality of peaks using a second method which differs from the first method in an algorithm or/and a parameter for extracting a peak for the mass chromatogram data of the reference samples, the mass chromatogram data being determined to have absence of a peak;
determining absence of a peak by collating the peak information extracted using the second method with the peak information of the standard sample; and
acquiring a feature amount corresponding to each of the plurality of peaks from the mass chromatogram data that is extracted using the first method or the second method, and is determined to have no absence of a peak, to create learning data.

2. The learning data creation method according to claim 1, wherein the mass chromatogram data that is determined to have absence of a peak in the peak information extracted using the first method or the second method is displayed on a screen.

3. The learning data creation method according to claim 2, wherein peak information is extracted using the second method for mass chromatogram data designated by a user among the mass chromatogram data displayed on the screen.

4. The learning data creation method according to claim 1, wherein
the mass chromatogram data is acquired for each of a plurality of reference samples having a same attribute,
mass chromatogram data of a plurality of reference samples having the same attribute are integrated to each other to create integrated mass chromatogram data, and
one acquired by extracting peak information related to a plurality of peaks included in the integrated mass chromatogram data using the first method is used as the peak information of the standard sample.

5. The learning data creation method according to claim 1, wherein processing of extracting peak information related to the plurality of peaks using a method having a different algorithm or/and a different parameter to determine absence of a peak is repeated a predetermined number of times until it is determined that there is no absence of a peak.

6. The learning data creation method according to claim 1, wherein
the first method and/or the second method extract(s), from among a plurality of measurement points constituting the mass chromatogram data, one having a measurement intensity that is lower than the measurement intensities of both adj acent measurement points, as a minimum measurement point,
determine(s) a baseline among the plurality of measurement points using the minimum measurement point, and
extract(s) a peak based on a fact that, at each of the plurality of measurement points, a value acquired by subtracting the baseline from the measurement intensity of the measurement point exceeds a threshold determined in advance.

7. The learning data creation method according to claim 6, wherein both the first method and the second method determine a baseline using the minimum measurement point to extract a peak, and the first method and the second method differ from each other in the threshold.

8. A learning data creation device for creating learning data to be used for creating a discriminator that discriminates a plurality of target samples having attributes that differ from each other, the device comprising:
a measurement data acquisition unit configured to acquire mass chromatogram data through measurement using a chromatograph mass spectrometer for each of a plurality of reference samples having known attributes;
a standard sample information storage unit configured to store peak information included in a mass chromatogram of a standard sample which has a same attribute as each of the reference samples;
a method information storage unit configured to store information of a first method for extracting a peak from the mass chromatogram data and a second method that differs from the first method in an algorithm or/and a parameter for extracting a peak;
a first peak extraction unit configured to extract peak information related to the plurality of peaks using the first method for the mass chromatogram data of each of the plurality of reference samples;
a first determination unit configured to determine absence of a peak by collating the peak information extracted by the first peak extraction unit with the peak information of the standard sample having a same attribute as each of the plurality of reference samples;
a second peak extraction unit configured to extract peak information related to the plurality of peaks using the second method for the mass chromatogram data of the reference samples, the mass chromatogram data being determined to have absence of a peak by the first determination unit;
a second determination unit configured to determine absence of a peak by collating the peak information extracted using the second method with the peak information of the standard sample; and
a learning data creation unit configured to acquire a feature amount corresponding to each of the plurality of peaks from the mass chromatogram data determined to have no absence of a peak by the first determination unit or the second determination unit to create learning data.

## Patentansprüche

1. Lerndatenstellungsverfahren zum Erstellen von Lerndaten, die zum Erstellen eines Diskriminators verwendet werden sollen, der eine Vielzahl von Zielproben, die Attribute aufweisen, die sich voneinander unterscheiden, basierend auf einer Merkmalsmenge in Bezug auf einen Peak diskriminiert, der in einem Massenchromatogramm jeder Zielprobe eingeschlossen ist, das Verfahren umfassend ein Durchführen, durch einen Computer, folgender Schritte:
Erfassen von Massenchromatogrammdaten über eine Messung unter Verwendung eines Chromatograph-Massenspektrometers für jede einer Vielzahl von Referenzproben, die bekannte Eigenschaften aufweisen;
Extrahieren von Peakinformationen, die sich auf die Vielzahl von Peaks beziehen, unter Verwendung eines ersten Verfahrens, das im Voraus vorbereitet wird, für die Massenchromatogrammdaten jeder der Vielzahl von Referenzproben;
Bestimmen eines Fehlens eines Peaks durch Vergleichen der Peakinformationen, die unter Verwendung des ersten Verfahrens extrahiert werden, mit Peakinformationen, die im Voraus einer Standardprobe, die ein gleiches Attribut wie jede der Vielzahl von Referenzproben aufweist, vorbereitet werden;
Extrahieren von Peakinformationen, die sich auf die Vielzahl von Peaks beziehen, unter Verwendung eines zweiten Verfahrens, das sich von dem ersten Verfahren in einem Algorithmus oder/und einem Parameter zum Extrahieren eines Peaks für die Massenchromatogrammdaten der Referenzproben unterscheidet, wobei für die Massenchromatogrammdaten bestimmt wird, dass sie das Fehlen eines Peaks aufweisen;
Bestimmen des Fehlens eines Peaks durch Vergleichen der Peakinformationen, die unter Verwendung des zweiten Verfahrens extrahiert werden, mit den Peakinformationen der Standardprobe; und
Erfassen einer Merkmalsmenge entsprechend jedem der Vielzahl von Peaks aus den Massenchromatogrammdaten, die unter Verwendung des ersten Verfahrens oder des zweiten Verfahrens extrahiert werden und für die bestimmt wird, dass sie kein Fehlen eines Peaks aufweisen, um Lerndaten zu erstellen.

2. Lerndatenstellungsverfahren nach Anspruch 1, wobei die Massenchromatogrammdaten, für die bestimmt wird, dass sie das Fehlen eines Peaks in den Peakinformationen aufweisen, die unter Verwendung des ersten Verfahrens oder des zweiten Verfahrens extrahiert werden, auf einem Bildschirm angezeigt werden.

3. Lerndatenstellungsverfahren nach Anspruch 2, wobei Peakinformationen unter Verwendung des zweiten Verfahrens für Massenchromatogrammdaten extrahiert werden, die durch einen Benutzer unter den Massenchromatogrammdaten, die auf dem Bildschirm angezeigt werden, designiert werden.

4. Lerndatenstellungsverfahren nach Anspruch 1, wobei
die Massenchromatogrammdaten für jede einer Vielzahl von Referenzproben, die ein gleiches Attribut aufweisen, erfasst werden,
Massenchromatogrammdaten einer Vielzahl von Referenzproben, die das gleiche Attribut aufweisen, ineinander integriert werden, um integrierte Massenchromatogrammdaten zu erstellen, und
die, die durch Extrahieren von Peakinformationen, die sich auf eine Vielzahl von Peaks beziehen, die in den integrierten Massenchromatogrammdaten eingeschlossen sind, unter Verwendung des ersten Verfahrens erfasst werden, als die Peakinformationen der Standardprobe verwendet werden.

5. Lerndatenstellungsverfahren nach Anspruch 1, wobei ein Verarbeiten des Extrahierens von Peakinformationen, die sich auf die Vielzahl von Peaks beziehen, unter Verwendung eines Verfahrens, das einen unterschiedlichen Algorithmus oder/und einen unterschiedlichen Parameter aufweist, um das Fehlen eines Peaks zu bestimmen, eine zuvor bestimmte Anzahl von Malen wiederholt wird, bis bestimmt wird, dass kein Fehlen eines Peaks vorliegt.

6. Lerndatenstellungsverfahren nach Anspruch 1, wobei
das erste Verfahren und/oder das zweite Verfahren aus einer Vielzahl von Messpunkten, die die Massenchromatogrammdaten bilden, einen, der eine Messintensität aufweist, die niedriger als die Messintensitäten beider angrenzender Messpunkte ist, als einen minimalen Messpunkt extrahiert/extrahieren,
eine Basislinie unter der Vielzahl von Messpunkten unter Verwendung des minimalen Messpunkts bestimmt/bestimmen, und
einen Peak basierend auf einer Tatsache extrahiert/extrahieren, dass an jedem der Vielzahl von Messpunkten ein Wert, der durch Subtrahieren der Basislinie von der Messintensität des Messpunkts erhalten wird, eine Schwelle, die im Voraus bestimmt wird, überschreitet.

7. Lerndatenstellungsverfahren nach Anspruch 6, wobei sowohl das erste Verfahren als auch das zweite Verfahren eine Basislinie unter Verwendung des minimalen Messpunkts bestimmen, um einen Peak zu extrahieren, und sich das erste Verfahren und das zweite Verfahren voneinander in der Schwelle unterscheiden.

8. Lerndatenstellungsvorrichtung zum Erstellen von Lerndaten, die zum Erstellen eines Diskriminators verwendet werden sollen, der eine Vielzahl von Zielproben diskriminiert, die Attribute aufweisen, die sich voneinander unterscheiden, die Vorrichtung umfassend:
eine Messdatenerfassungseinheit, die konfiguriert ist, um Massenchromatogrammdaten über die Messung unter Verwendung eines Chromatograph-Massenspektrometers für jede einer Vielzahl von Referenzproben, die bekannte Eigenschaften aufweisen, zu erfassen;
eine Standardprobeninformationsspeichereinheit, die konfiguriert ist, um Peakinformationen zu speichern, die in einem Massenchromatogramm einer Standardprobe, die ein gleiches Attribut wie jede der Referenzproben aufweist, eingeschlossen sind;
eine Verfahrensinformationsspeichereinheit, die konfiguriert ist, um Informationen eines ersten Verfahrens zum Extrahieren eines Peaks aus den Massenchromatogrammdaten und eines zweiten Verfahrens, das sich von dem ersten Verfahren in einem Algorithmus oder/und einem Parameter zum Extrahieren eines Peaks unterscheidet, zu speichern;
eine erste Spitzenextraktionseinheit, die konfiguriert ist, um Peakinformationen, die sich auf die Vielzahl von Peaks beziehen, unter Verwendung des ersten Verfahrens für die Massenchromatogrammdaten jeder der Vielzahl von Referenzproben zu extrahieren;
eine erste Bestimmungseinheit, die konfiguriert ist, um das Fehlen eines Peaks durch Vergleichen der Peakinformationen, die durch die erste Peakextraktionseinheit extrahiert werden, mit den Peakinformationen der Standardprobe, die ein gleiches Attribut wie jede der Vielzahl von Referenzproben aufweist, zu bestimmen;
eine zweite Peakextraktionseinheit, die konfiguriert ist, um Peakinformationen, die sich auf die Vielzahl von Peaks beziehen, unter Verwendung des zweiten Verfahrens für die Massenchromatogrammdaten der Referenzproben zu extrahieren, wobei für die Massenchromatogrammdaten durch die erste Bestimmungseinheit bestimmt wird, dass sie das Fehlen eines Peaks aufweisen;
eine zweite Bestimmungseinheit, die konfiguriert ist, um das Fehlen eines Peaks durch Vergleichen der Peakinformationen, die unter Verwendung des zweiten Verfahrens extrahiert werden, mit den Peakinformationen der Standardprobe zu bestimmen; und
eine Lerndatenerstellungseinheit, die konfiguriert ist, um eine Merkmalsmenge entsprechend jedem der Vielzahl von Peaks aus den Massenchromatogrammdaten zu erfassen, für die durch die erste Bestimmungseinheit oder die zweite Bestimmungseinheit bestimmt wurde, dass sie kein Fehlen eines Peaks aufweisen, um Lerndaten zu erstellen.

## Revendications

1. Procédé de création de données d'apprentissage destiné à créer des données d'apprentissage à utiliser pour créer un discriminateur qui discrimine une pluralité d'échantillons cibles ayant des attributs qui diffèrent les uns des autres en fonction d'une quantité de caractéristiques par rapport à un pic inclus dans un chromatogramme de masse de chaque échantillon cible, le procédé comprenant la réalisation par ordinateur des étapes suivantes consistant à :
acquérir des données de chromatogramme de masse par une mesure à l'aide un spectromètre de masse chromatographe pour chacun d'une pluralité d'échantillons de référence ayant des attributs connus ;
extraire des informations sur les pics en lien avec la pluralité de pics à l'aide d'un premier procédé préparé à l'avance pour les données de chromatogramme de masse de chacun de la pluralité d'échantillons de référence ;
déterminer l'absence d'un pic en rassemblant les informations sur les pics extraites à l'aide du premier procédé avec des informations sur les pics qui sont préparées à l'avance pour un échantillon standard qui a le même attribut que chacun de la pluralité d'échantillons de référence ;
extraire des informations sur les pics en lien avec la pluralité de pics à l'aide d'un second procédé qui diffère du premier procédé dans un algorithme ou/et un paramètre permettant d'extraire un pic pour les données de chromatogramme de masse des échantillons de référence, les données de chromatogramme de masse étant déterminées comme étant dépourvues de pic ;
déterminer l'absence d'un pic en rassemblant les informations sur les pics extraites à l'aide du second procédé avec les informations sur les pics de l'échantillon standard ; et
acquérir une quantité de caractéristiques correspondant à chacun de la pluralité de pics à partir des données de chromatogramme de masse qui sont extraites à l'aide du premier procédé ou du second procédé, et dont il est déterminé qu'elles ne sont pas dépourvues de pic, afin de créer des données d'apprentissage.

2. Procédé de création de données d'apprentissage selon la revendication 1, dans lequel les données de chromatogramme de masse qui sont déterminées comme dépourvues d'un pic dans les informations sur les pics extraites à l'aide du premier procédé ou du second procédé sont affichées sur un écran.

3. Procédé de création de données d'apprentissage selon la revendication 2, dans lequel des informations sur les pics sont extraites à l'aide du second procédé pour des données de chromatogramme de masse désignées par un utilisateur parmi les données de chromatogramme de masse affichées sur l'écran.

4. Procédé de création de données d'apprentissage selon la revendication 1, dans lequel
les données de chromatogramme de masse sont acquises pour chacun d'une pluralité d'échantillons de référence ayant un même attribut,
des données de chromatogramme de masse d'une pluralité d'échantillons de référence ayant le même attribut sont intégrées les unes aux autres pour créer des données de chromatogramme de masse intégrées, et
l'une d'entre elles, acquise lors de l'extraction d'informations sur les pics relatives à une pluralité de pics inclus dans les données de chromatogramme de masse intégré à l'aide du premier procédé, est utilisée comme information sur un pic de l'échantillon standard.

5. Procédé de création de données d'apprentissage selon la revendication 1, dans lequel le traitement d'extraction d'informations sur les pics en lien avec la pluralité de pics à l'aide d'un procédé ayant un algorithme différent ou/et un paramètre différent pour déterminer l'absence d'un pic est répété un nombre prédéterminé de fois jusqu'à ce qu'il soit déterminé qu'il n'y a pas d'absence de pic.

6. Procédé de création de données d'apprentissage selon la revendication 1, dans lequel
le ou les extraits du premier procédé et/ou du second procédé, parmi une pluralité de points de mesure constituant les données de chromatogramme de masse, l'un ayant une intensité de mesure qui est inférieure aux intensités de mesure des deux points de mesure adjacents, en tant que point de mesure minimum,
détermine(nt) une ligne de base parmi la pluralité de points de mesure à l'aide du point de mesure minimum, et
extrai(en)t un pic sur la base du fait que, à chacun de la pluralité de points de mesure, une valeur acquise en soustrayant la ligne de base de l'intensité de mesure du point de mesure dépasse un seuil déterminé à l'avance.

7. Procédé de création de données d'apprentissage selon la revendication 6, dans lequel à la fois le premier procédé et le second procédé déterminent une ligne de base à l'aide du point de mesure minimum pour extraire un pic, et le premier procédé et le second procédé diffèrent l'un de l'autre par le seuil.

8. Dispositif de création de données d'apprentissage permettant de créer des données d'apprentissage à utiliser pour créer un discriminateur qui discrimine une pluralité d'échantillons cibles ayant des attributs qui diffèrent les uns des autres, le dispositif comprenant :
une unité d'acquisition des données de mesure conçue pour acquérir des données de chromatogramme de masse par mesure à l'aide d'un spectromètre de masse chromatographe pour chacun d'une pluralité d'échantillons de référence ayant des attributs connus ;
une unité de stockage d'informations d'échantillon standard conçue pour stocker des informations sur les pics incluses dans un chromatogramme de masse d'un échantillon standard qui a un même attribut que chacun des échantillons de référence ;
une unité de stockage d'informations de procédé conçue pour stocker des informations d'un premier procédé d'extraction d'un pic à partir des données de chromatogramme de masse et d'un second procédé qui diffère du premier procédé en ce qui concerne un algorithme ou/et un paramètre permettant d'extraire un pic ;
une première unité d'extraction de pics conçue pour extraire des informations sur les pics relatives à la pluralité de pics à l'aide du premier procédé pour les données de chromatogramme de masse de chacun de la pluralité d'échantillons de référence ;
une première unité de détermination conçue pour déterminer l'absence d'un pic en rassemblant les informations sur les pics extraites par la première unité d'extraction des pics avec les informations sur les pics de l'échantillon standard ayant un même attribut que chacun de la pluralité d'échantillons de référence ;
une seconde unité d'extraction de pic conçue pour extraire des informations sur les pics en lien avec la pluralité de pics à l'aide du second procédé pour les données de chromatogramme de masse des échantillons de référence, les données de chromatogramme de masse étant déterminées comme étant dépourvues de pic par la première unité de détermination ;
une seconde unité de détermination conçue pour déterminer l'absence d'un pic en rassemblant les informations sur les pics extraites à l'aide du second procédé avec les informations sur les pics de l'échantillon standard ; et
une unité de création de données d'apprentissage conçue pour acquérir une quantité de caractéristiques correspondant à chacun de la pluralité de pics à partir des données de chromatogramme de masse déterminées comme non dépourvues de pic par la première unité de détermination ou la seconde unité de détermination afin de créer des données d'apprentissage.
